# EUROPÄISCHE PATENTSCHRIFT

(11) **EP 0 553 167 B1**
(45) Veröffentlichungstag und Bekanntmachung des Hinweises auf die Patenterteilung: **04.03.1998**
(21) Anmeldenummer: 91918051.3
(22) Anmeldetag: 21.10.1991
(51) Int. Cl.: A61F 2/28, C04B 38/04, C08J 9/26, A61L 27/00, B01D 39/00

(54) **WERKSTOFF UND VERFAHREN ZU SEINER HERSTELLUNG**
MATERIAL AND PROCESS FOR PRODUCING THE SAME
MATERIAU ET SON PROCEDE DE PRODUCTION

(30) Priorität: 19.10.1990 DE 4033291
(43) Veröffentlichungstag der Anmeldung: 04.08.1993
(60) Teilanmeldung: 97108068.4
(73) Patentinhaber: Draenert, Klaus, Dr.med.Dr.med.habil., D-81545 München (DE)
(72) Erfinder: Draenert, Klaus, Dr.med.Dr.med.habil., D-81545 München (DE)
(86) Internationale Anmeldenummer: EP9101997
(87) Internationale Veröffentlichungsnummer: WO9206653

(56) Entgegenhaltungen:
- EP-A- 0 107 476
- EP-A- 0 277 678
- EP-A- 0 338 981
- WO-A-89/00842
- DE-A- 2 242 867
- DE-A- 3 309 855
- DE-A- 3 531 144
- GB-A- 2 093 701
- US-A- 4 645 503
- US-A- 4 859 712
- US-A- 4 891 182

## Beschreibung

Die Erfindung betrifft einen Verbundwerkstoff , ein Verfahren zu seiner Herstellung und seine Verwendung .

Es besteht allgemein und insbesondere in der Medizin ein Bedürfnis nach Verbundwerkstoffen , die unter grösstmöglicher Materialeinsparung und/oder bei niedrigem Gewicht ein Höchstmass an Festigkeit bieten . Es besteht insbesondere ein Bedürfnis nach Werkstoffen mit einem einstelllbaren Hohlraumsystemn mit interkonnektierenden Poren .

Aus der DE-C2-3106917 ist ein Verfahren zur Herstellung eines Implantates als Knochenersatz in Form eines offenporigen bzw. offenzelligen Formkörpers aus körpervertraeglichem Metall bekannt , wobei das Metall unter Anwendung eines verlorenen Modells verarbeitet wird . Bei diesem Verfahren werden als Positivmodell offenporige bzw. offenzellige Natur-oder Kunstschwämme mit einer durchschnittlichen Weite der Poren oder Zellen zwischen 0,5 und 1,5 mm verwendet , die mit einer keramischen Einbettmasse gefüllt werden . Anschliessend wird das Modellmaterial durch Hitze zerstört und entfernt , wodurch ein Keramik-Negativmodell entsteht . Danach werden die zuvor von dem Material des Positivmodells , d.h. dem Natur- oder Kunstschwamm , eingenommenen Räume durch ein giess- oder schleuderbares Metall gefüllt und anschliessend wird das Keramikmaterial des Negativmodells wieder entfernt . Ein Nachteil dieses Verfahrens besteht darin ,dass die Struktur des Positivmodells und damit die Struktur des fertigen Metallimplantates nicht gezielt einstellbar ist , sondern dass die Struktur jeweils so akzeptiert werden muss , wie die Schwämme , seien es Natur- oder Kunstschwämme , beschaffen sind . Ein weiterer Nachteil dieses Verfahrens besteht darin , dass es umständlich ist und zwei Formkörper , d.h. zunächst ein Positivmodell und danach ein Negativmodell , benötigt werden , um letztendlich ein dreidimensionales Gerüst zu schaffen .

In der DE-A-2910627 wird vorgeschlagen , ein aus Fäden oder Fasern aus Kunststoff oder Metall aufgebautes formstabiles Netzwerk vorzugeben und dieses als Implantat zu verwenden . Dieser Vorschlag ist deswegen schwer realisierbar , da die Netzwerke sehr schwierig und teuer herzustellen sind und es kaum gelingt , die Formstabilität reproduzierbar sicherzustellen und eine entsprechende Tragfähigkeit des Implantates zu garantieren .

In der OFFENLEGUNGSSCHRIFT 2242867 wird ein Verfahren zur Herstellung implantierbarer proröser , keramischer Knochenersatz-, Knochenverbund- oder Prothesenverankerungswerkstoffe vorgestellt , bei dem im eindrucksvollsten Beispiel ein Anlösen von Formkörpern aus Styropor vorgeschlagen wird , was jedoch dazu führt , dass sich , wie dies auch beschrieben ist , zwischen den Styroporkugeln als Formkörper ein scheibenförmiger Meniskus des Lösungsmediums bildet , der die intertrabekulären Räume weitgehend und undefinierbar verlegt. Ähnlich unregelmässige , meist scheibenförmige Verschmelzungen bilden sich bei niedrigschmelzenden Füllkörpern und Klebern , wodurch sich keine regelmässigen tragfähigen Fachwerke erzeugen lassen . Auch lastet dem Verfahren an , dass die gesamten Füllkörper durch Hitze verbrannt werden , was die Implantate kontaminiert . Der Hauptmangel liegt in der fehlenden Einstellbarkeit und Gleichförmigkeit , sowie der fehlenden Steuerbarkeit der interkonnektierenden Porosität .

Der Erfindung liegt somit die Aufgabe zugrunde , einen Verbundwerkstoff und ein Verfahren zu seiner Herstellung bereitzustellen , die es ermöglichen , ein dreidimensionales Gerüst sowohl im Hinblick auf die Porosität des Werkstoffes und die Dicke der tragenden Struzkturen als auch im Hinblick auf die Formstabilität und andere gewünschter Eigenschaften , wie Löslichkeit oder Resorbierbarkeit , exakt nach den jeweiligen Bedürfnissen einzustellen .

Diese Aufgabe wird durch die vorliegende Erfindung gemäss den Patentansprüchen gelöst . Die erfindung löst somit ein altes Problem in der Werkstoffherstellung , das insbesondere für die Medizin sehr grosse Bedeutung hat , nämlich die Herstellung von durchgehend porösen Gerüsten mit einer einstellbaren Porosität und einer einstellbaren Festigkeit sowie jeweils den Bedürfnissen angepassten Materialeigenschaften , wie Löslichkeit und Resorbierbarkeit . Dabei wird vorzugsweise jeweils von einem Negativmodell des fertigen Werkstoffs ausgegangen .

Die Erfindung geht von dem Grundgedanken aus , miteinander verbundene einzelne Formkörper , z.B. in Form einer dichten Packung oder eines Konmglomerats , als Platzhalter für die Hohlraumstruktur zu verwenden und um die leicht entfernbaren , beispielsweise leicht löslichen oder leicht schmelzbaren Formkörper herum ein dreidimensionales Gerüst aus tragenden Strukturen zu schaffen , vorzugsweise aus einem giessfähigen Material. Im Anschluss daran können die Formkörper entweder physikalisch oder chemisch , vorzugsweise durch Anwendung des Prinzips der Wasserlöslichkeit oder der Schmelzbarkeit , oder durch Anwendung von Hitze wieder entfernt werden . Erfindungsgemäss können auch die einzelnen Formkörper selbst im Sinne eines Positiv-Negativmodells hergestellt werden .

Anders ausgedrückt beruht die Erfindung im Prinzip darauf , dass durch Formkörper vorgegebene Konfiguration , beispielsweise kugelförmige oder granuläre Formkörper , welche punktförmig miteinander zu einer dreidimensional Struktur in Form eines Konglomerates verbunden werden , eine durchgehende bälkchenförmige oder trabekuläre Hohlraumstruktur entsteht ; diese Hohlraumstruktur wird anschliessend mit einem anderen Material ausgefüllt. Die räumliche Struktur des zum Ausfüllen verwendeten Materials stellt das "Positivmodell" zu dem von dem Formkörperkonglomerat gebildeten "Negativmodell" dar und ist durch geeignete Wahl der Struktur des Negativemodells beliebig einstellbar . Der nach dem Ausfüllen entstandene Verbundkörper kann anschliessen von den ein "Inlet" bildenden Formkörper befreit werden . Durch die Entfernung der Formkörper entsteht ein durchgehend poröser Werkstoff . Die Porosität des Werkstoffes ist durch geeignete Auswahl der Grösse , Grössenverteilung und Schüttdichte der Formkörper sowie der Art deren gegenseitiger Verbindung reproduzierbar einstellbar . Der fertige Werkstoff ist mechanisch widerstandsfähig und kann in seiner äusseren Form beliebig gestaltet werden .

Der erfindungsgemässe Werkstoff unterscheidet sich von mit herkömmlichen Verfahren hergestellten Werkstoffen, beispielsweise aus der Zahntechnik dadurch , dass sich sowohl die Porosität als auch die Stärke der trabekelartigen tragenden Strukturen und die Festigkeit exakt einstellen lassen .

Durch Sinterverfahren können erfindungsgemäss Keramik- oder Keramikverbundwerkstoffe hoher Dichte sowohl als Positiv- als auch als Negativformkörper hergestellt werden . Es können erfindungsgemäss auch mit giessbaren Metallen oder Metallverbundwerkstoffen hochdicht Werkstoffe in Foirm von Metalltrabekeln oder Metallbälkchen mit durchgehender Poirosität , d.h. mit miteinander in Verbindung stehenden Hohlräumen , hergestellt werden; derartige Werkstoffe sind für die Technik von grösstem Interesse .

Der erfindungsgemässe Werkstoff findet insbesondere in der Medizintechnik und dort ganz besonders für die Herstellung von Implantaten oder auch als Wirkstofftraeger in sogenannten "drug delivery"-Systemen Verwendung . Der erfindungsgemässe Werkstoff kann aber auch als Träger für alle diejenigen Wirkstoffe verwendet werden , die auf Oberflächen aufgetragen werden können und zu denen beispielsweise das " bone morphogenetic protein" oder gewisse Wachstumsfaktoren zählen .

Entlang der vorzugsweise beschichteten Oberflächen der vorgebbar einstellbaren Hohlraumstruktur kommt es erwiesenermassen sehr schnell zu einem gezielten Knocheneinwuchs , der bei Verwendung des erfindungsgemässen Werkstoffs als Implantat auch unabhängig von der biomechanischen Beanspruchung knöchern durchwachsen kann .

Im Prinzip können alle beliebigenn Werkstoffe in die Negativ- oder Positivform gebracht werden , indem beispielsweise wasserlösliche und säurelösliche Formkörper mit schmelzbaren Materialien oder wasserlösliche oder säurelösliche Formkörper mit sintzerfähigen , giessbaren Materialverbunden oder im Spritzgussverfahren verarbeitbaren Kunststoffen oder giessbaren , beispielsweise im Schleuderguss oder Sprittzguss verarbeitbaren Metallen , Metallegierungen oder Metallverbundwerkstoffen in der Weise kombiniert werden , dass jeweils eine Sorte Formkörper , entweder physikalisch oder chemisch oder auf andere Weise , wieder herauslösbar ist und das dreidimensiomale Gerüst als Stützgerüst verbleibt . Das Stützgersüst kann anschliessend durch physikalische oder chemische Verfahren verfestigt , oberflächenbehandelt oder mechanisch nachbearbeitet werden .

Die Formkörper und das vorzugsweise aus einem giessbaren Material bestehende Stützgerüst können auch zusammen , beispielsweise mechanisch , weiterverarbeitet werden und erst im Anschluss daren physikalisch oder chemisch separiert werden . Die so erhaltenen dreidimensionalen Gerüste können wiederum temporär als Formkörper für Negativmodelle dienen , sodass sowohl das dreidimensionale Gerüst mit durchgehender Porosität als auch die Packung bzw. das Konglomerat aus miteinander verbundenen und vorzugsweise kugelförmigen Formkörpern wechselseitig als Modell eingesetzt werden können . Auf diese Weise können alle möglichen Materialien , Materialkombinationen und Verbundwerkstoffe zu dem erfindungsgemässen Werkstoff verarbeitet werden .

Das Material für das dreidimensionale Gerüst ist vorzugsweise giessbar oder spritzbar , beispielsweise im Spritzgussverfahren . Falls der Werkstoff als Implantat verwendet wird , besteht das dreidimensionale Gerüst des erfindungsgemässen Werkstoffes vorzugsweise aus einem Polymer auf der Basis eines Polyacrylates oder eines Polymetacrylates , einem Copolymer eines Acrylates und Metacrylates , einer Mischung aus diesen oder aus einem anderen körpervertraeglichen Kuinststoff . Das dreidimensioonale Gerüst kann auch aus einer resorbierbaren Polyaminosäure , einem Polylactat , einem Polyglykolat , einem Gemisch aus verschiedenen Polyaminosäuren oder einem anderen im Körper auflösbaren und/oder resorbierbaren Material bestehen . Silikon oder ein Kautschukderivat kommen ebenfalls als Werkstoff in Betracht .

Die als Platzlhalter für das Hohlraumsystem des Werkstoffes dienenden Formkörper weisen vorzugsweise die Form von Kugeln oder gleichmässigen geometrischen Körpern , beispielsweise Vielecken auf , es kann aber auch Granulatmaterial als Formkörper verwendet werden . Vorzugsweise ist das Material der Formkörper leicht löslich, z.B. wasserlöslich oder säurelöslich, oder leicht schmelzbar . Besonders bevorzugt sind Formkörper aus einem wasserlöslichen Material , beispielsweise aus Zucker , die zunächst im Wasserdampf zur Bildung eines Formkörperkonglomerats fest miteinander vertklebt werden können und nach der Ausbildung und Aushärtung des dreidimensionalen Gerüstes ausgewaschen werden können , beispielsweise im Wasserbad oder in der Waschmaschine .

Als Material für die Formkörper kann ein anorganischer oder keramischer Werkstoff , belspielsweise Tricalciumphosphat , ein Hydroxylapatit , ein Gemisch aus beiden oder eine andere , leicht oder schwer resorbierbare Calciumverbindung verwendet werden , insbesondere wenn der Werkstoff für ein Implantat verwendet wird . Bevorzugt sind säurelösliche keramische Werkstoffe.

Da sowohl die Grösse als auch die Schüttdichte und die Art der gegenseitigen Verbindung der als Platzhalter für das Hohlraumsystem des Werkstoffes dienenden Formkörper frei wählbar ist , ist auch das Hohlraumsystem des fertigen Werkstoffes bezüglich seiner Porosität frei einstellbar . Beispielsweise können kugelförmige Formkörper verwendet werden , wenn ein Hohlraumsystem mit im wesentlichen kugelförmigen und miteinander in Verbindung stehenden Hohlräumen angestrebt wird . Wenn ein Hohlraumsystem mit unterschiedlich grossen Hohlräumen angestrebt wird , können Formkörper unterschiedlicher Grösse und/oder Form miteinander gemischt werden . Die gesamte Porosität des fertigen Werkstoffes ist durch Einstellung , Variation und Kombination der Form und/oder Schüttdichte der Formkörper und/oder Wahl des Verfahrens , durch das die Formkörper zu einem Konglomerat miteinander verbunden werden, gezielt einstellbar.

Die Form und Gestaltung der Bälkchen bzw. Trabekel des dreidimensionalen Gerüstes ist erfindungsgemäss ebenso vorgebbar und gezielt je nach Verwendungszweck einstellbar .

Die Formkörper können beispielsweise durch ein Sinterverfahren miteinander verbunden werden . Die bevorzugte Grösse der Formkörper betraegt zwischen etwa 0,5 und 5mm , besonders bevorzugt sind 3 mm , insbesondere falls der Werkstoff als Implantat verwandt wird .

Das HIP-Verfahren stellt eine bevorzugte Form dar , wobei das Geruestwerk zusammen mit den Formkörpern verdichtet wird , um eine weitere Variation der gewünschten Eigenschaften des fertigen Werkstoffes zu ermöglichen .

Der Verbundwerkstoff kann auch zusätzlich Füllerpartikel enthalten, beispielsweise Tricalciumphosphat oder Hydroxylapatit oder ein Gemisch aus beiden mit einem Anteil von 1 bis 95% , vorzugsweise 1 bis 80% , und mit einer Partikelgrösse von 50 bis 300 µm , besonders bevorzugt bis 250 µm , und einem Porenvolumen von 0,1 ml/g bis 0,8ml/g.

Je nach der beabsichtigten Verwendung können dem Werkstoff auch verschiedene Wirkstoffe zugesetzt werden , beispielsweise kann das Material für das dreidimensional Gerüst 0,01 bis 10% eines Wirkstoffes enthalten , der aus dem Werkstoff verzögertt freigesetzt werden kann . Als Wirkstoffe kommen bei Verwendung des Verbundwerkstoffes als Implantat beispielsweise Antibiotika , ein das Knochenwachstum induzierender Wirkstoff , ein Wachstumsfaktor oder ein anderer chemotaktisch oder hormonell wirkender Faktor in Betracht , der das Einsprossen der Gefaesse oder direkt die Stimulation der Osteoblastem bewirkt .

Die äussere Form des Verbundwerkstoffes ist frei wählbar und richtet sich nach dem jeweiligen Verwendungszweck .

### Beispiel 1

### Herstellen eines porösen Implantates mit hoher mechanischer Festigkeit

Keramikkugeln mit einem Durchmesser zwischen 0,5 und 3mm werden in einem Sinterofen zusammengesintert und punktförmig zu einem Konglomerat in Form eines porösem Körpers verschweisst , der als Negativmodell dient . Die miteinander in Verbindung stehenden bzw. interkonnektierenden Poren (Hohlräume) des Konglomerates werden mit einem giessfähigen Metall im Schleudergussverfahren ausgefüllt . Der so entstandene Verbundwerkstoff aus Keramik und Metall wird anschliessend im sogenannten "HIP-Verfahren" sehr hoch verdichtet. Danach wird die Keramik auf chemischem Wege , beispielsweise durch Anwendung von Säure , wieder entfernt , und es verbleibt ein Werkstoff aus einem dreidimensionalen Metallgerüst mit tragenden bälkchenartigen Strukturen und einem interkonnektierenden Porensystem , das beispielsweise als Implantat verwendbar ist.

### Beispiel 2

### Herstellen eines hochfesten keramischen Implantatkörpers

Metallkugeln einer Grösse zwischen 0,5 und 3mm werden als Formkörper verwendet und durch Hitze oder punktförmige Verklebung miteinander verschweisst bzw. verbunden , sodass ein durchgehendes trabekuläres System von Hohlräumen um die Kugeln entsteht . Diese sehr festen Formkörper werden anschliessend mit einer keramischen Masse im Schleudergussverfahren umgossen . Danach wird der entastandene Verbundwerkstoff im "Hip-Verfahren" hoch verdichtet .Anschliessend werden die Metallkugeln auf elektrolytischem Wege herausgelöst . Das verbleibende dreidimensionale Keramikgerüst wirk im Sinterverfahren weiter verfestigt . Auf diese Weise wird ein hochfester keramischer Implantatkörper mit dreidimensionaler trabekulärer Struktu hergestellt . Die Poren des durchgehenden Hohlraumsystems sind durch geeignet Wahl der Grösse der als Platzhalter dienenden Metallkugeln einstellbar .

### Beispiel 3

### Herstellung eines als Prothesenschaft für eine Femurkomponente einer Hüftgelenksprotheses verwendbaren Implantatkörpers

In eine anatomisch geformte erste Form werden Keramikkugeln einer Grösse zwischen 0,5 und 1,5 mm lose aufgeschüttet und im Sinterofen zusammengesintert . Der erhaltene Körper (Konglomerat) aus gesintertem Material wirk anschliessend in einer ähnlich aufgebauten zweiten Form , die jedoch schalenförmig etwa 3 - 4 mm weiter ist als die erste Form , so versenkt , dass ein gleichförmiger Spalt um den gesinterten Körper verbleibt . Dieser Spalt wird mit Keramikkugeln einer Grösse von 1 -3 mm aufgefüllt . Anschliessend wird die gefüllte zweite Form in einem Sinterofen gesintert , sodass aus den beiden unterschiedlichen Kugelkomponenten ein Verbundkörper aus gesinterten Kugeln entsteht . Diese Verbundkörper wird als Modell im Schleudergussverfahren mit einer giessbaren Metallegierung ausgegossen und anschliessend im "Hip-Verfahren" so verdichtet , dass ein hochfester Metallkeramikverbund entsteht . Im Anschluss daran wird die Keramik bis auf eine Oberflächenbeschichtung des Metalles wieder entfernt , vorzugsweise auf chemischem Wege . Auf diese Weise entsteht ein hochfester , vollständig poröser Metallkörper , der im Bereich seiner Oberfläche unterschiedliche mechanische Eigenschaften gegenüber dem massiveren Innenteil mit dichterer Struzktur aufweist . Diese Oberfläche stellt bei Verwendung des Metallkörpers als Implantat oder Prothese die Grenzzone zum Knochen dar . Eine solche Schichtung kann auch in axialer Richtung in verschiedener Weise wiederholt eingestellt werden , so dass insgesamt jede beliebige Prothesenschaft-Konfiguration mechanisch einstellbar und reproduzierbar gefertigt werden kann . Die optimale Prothesenschaft-Konfiguration kann beispielsweise nach Finite-Elemente-Methoden errechnet werden . Es lassen sich somit auch sehr kompliziert aufgebaute Implantatkörper oder Prothesen mit einem durchgehenden inneren Hohlraumsystem beliebig und gezielt herstellen .

## Patentansprüche

1. Verbundwerkstoff , bestehend aus einem dreidimensionalen Gerüst aus tragenden bälkchenartigen Strukturen exakt einstellbarer Festigkeit und Dicke , Löslichkeit und/oder Resorbierbarkeit , die über exakt einstellbare interkonnektierende Poren in Verbindung stehende Formkörper umschliessen , wobei das dreidimensionale Gerüst zusammen mit den Formkörpern verdichtet ist .

2. Versbundwerkstoff nach Anspruch 1 , so beschaffen , dass die Formkörper und das Gerüst physikalisch oder chemisch so unterschiedliche Eigenschaften haben , dass die Formkörper teilweise oder ganz entfernbar sind , ohne das Gerüst zu gefährden .

3. Verbundwerkstoff nach den Anssprüchen 1-2 , so beschaffen , dass die Formkörper als eine variierbare dichte Kugelpackung einzelner Kugeln oder als Kugelkonglomerat aus einzelnen fest miteinander verbundenen Kugeln vorliegen .

4. Verbundwerkstoff nach den Ansprüchen 1-2 , so beschaffen , dass die Formkörper aus gleichmässigen geometrischen Körpern , Vielecken , Granulat oder aus einem Gemisch aus verschiedenen gleichförmigen oder ungleichförmigen Formkörpern bestehen und zwar in ausgewählt dichter Schüttung oder aus einem Konglomerat aus fest miteinander verbundenen Formkörpern .

5. Verbundwerkstoff nach den Abnsprüchen 1-4 , so beschaffen , dass die Formkörper chemisch , physikalisch , durch Schmelzen , Kleben , Sintern miteinander verbunden sind .

6. Verbundwerkstoff nach den Ansprüchen 1-5 , so beschaffen , dass als Material für Gerüst oder Formkörper Keramiken, Metalle, körpervertraegliche Kunststoff , leicht lösliche Substanzen wie Zucker , leicht schmelzbare Materialien wie Wachs , Silikon , gummiartige Polymere , resorbierbare Polymere, Polylactide, Polyaminosäuren, Polyglycide , spritzbare , v.a. auch wasserlösliche , giessbare , elektrolytisch bearbeitbare und/oder im Spritzgussverfahren verarbeitbare Werkstoffe eingesetzt werden .

7. Verbundwerkstoff nach Anspruch 6, so beschaffen , dass als Material für die Formkörper Tricalciumphosphat , Hydroxylapatit , ein Gemisch aus beiden oder eine andere leicht oder schwer resorbierbare Calciumverbindung verwendet wird .

8. Verbundwerkstoff nach Anspruch 6 , so beschaffen , dass als körpervertraegliche Kunststoffe Polymere auf der Basis eines Acrylates , Polyacrylates , Polymetacrylates oder eines anderen körpervertraeglichen, resorbierbaren oder nacht resorbierbaren Kunststoffes verwendet werden .

9. Verbundwerkstoff nach Anspruch 1-8 , so beschaffen , dass die Formkörper aus Keramik bestehen und das Gerüst aus Metallausguss besteht .

10. Verbundwerkstoff nach den Ansprüchen 1-8 , wobei die Formkörper aus einem leicht schmelzbaren Material, wie Wachs oder aus Zucker oder aus einem anderen löslichen, vorzugsweise wasserlöslichen Material bestehen oder wobei die Formkörper aus einem keramischen Werkstoff bestehen , vorzugsweise Tricalciumphosphat , einem Hydroxylapatit, einem Gemisch aus beiden oder aus einer anderen , leicht oder schwer resorbierbaren Calciumverbindung .

11. Verbundwerkstoff nach den Ansprüchen 1-10 , wobei die Formkörper im wesentlichen kugelförmig sind .

12. Verbundwerkstoff nach einem der Ansprüche 1-11 , wobei die Formkörper eine Grösse zwischen 0,2 und einigen mm , vorzugsweise 5mm aufweisen .

13. Verbundwerkstoff nach einem der Ansprüche 1-12 , wobei eine Mischung von Formkörpern verschiedener Grösse verwendet wird .

14. Verbundwerkstoff nach einem der Ansprüche 2 und 8 , wobei die Formkörper teilweise entfernt sind und das Gerüst einen Füller aufweist .

15. Verbundwerkstoff nach Anspruch 14 , wobei der Füller aus Partikeln besteht und als Füllerpartikel 1 bis 95% , vorzugsweise 1-80% Tricalciumphosphat oder Hydroxylapatit oder ein Gemisch aus beiden oder eine Calciumverbindung , vorzugsweise mit einer Partikelgrösse von 50 - 300µm , besonders bevorzugt bis zuu 250µm und einem Porenvolumen von 0,1 m l/g bis 0,8ml/g verwendet werden .

16. Verbundwerkstoff nach Anspruch 15 , worin die Ca-Verbindung eine Calciumphosphatverbindung ist .

17. Verfahren zum Herstellen eines Verbundwerkstoffes mit den folgenden Verfahrensschritten ;
Erzeugen einer dichten Packung von Formkörpern oder Verbinden von Formkörpern zu einem dreidimensionalen Formkörper-Konglomerat .
Formen eines sich von den Formkörpern unterscheiden Materials um die Formkörper herum zur Ausbildung eines dreidimensionalen Gerüstes .
Verdichten des Verbundkörpers aus Formkörpern und Gerüstrstoff . Ganz oder teilweises Entfernen der Formkörper , ohne dass das Gerüst zerestört wird .

18. Verfahren nach Anspruch 17 , wobei die Formkörper kugelförmig sind .

19. Verfahren nach Anspruch 17 und 18 , wobei die Formkörper punktförmig miteinander verbunden werden .

20. Verfahren nach Anspruch 17-18 , wobei die Formkörper chemisch oder pohysikalisch miteinander verschweisst oder fest miteinander verklebt werden , beispielsweise im Wasserdampf .

21. Verfahren nach einem der Ansprüche 17-21 , wobei als Material für das dreidimensionale Gerüst ein Giess-oder Spritzgusswerkstoff verwendet wird und die Formkörper nach dem Aushärten dieses Materials ausgewaschen werden , beispielsweise im Wasserbad oder in der Waschmaschine .

22. Verfahren nach einem der Ansprüche 17-22 , wobei die Formkörper im Sinterverfahren miteinander verbunden werden .

23. Verfahren nach Anspruch 17 , wobei nach vollständigem Entfernen der Formkörper das verbleibende Hohlraumsystem mit Metall ausgegossen wird .

## Claims

1. A composite material, comprising a three-dimensional framework of supportive trabecular structures of exactly definable strength and density, solubility and/or resorbability, which encompass shaped bodies that communicate via exactly definable interconnecting pores, the three-dimensional framework together with the shaped bodies being compressed.

2. A composite material according to claim 1, made such that the shaped bodies and the framework have such different properties physically or chemically that the shaped bodies can be removed in whole or in part without endangering the framework.

3. A composite material according to claims 1-2, made such that the shaped bodies exist in the form of a variably dense package of individual spheres or as a conglomerate of individual, firmly connected spheres.

4. A composite material according to claims 1-2, made such that the shaped bodies consist of uniform geometrical bodies, polygons, granulate or a mixture of different uniform or non-uniform shaped bodies, especially in a chosen dense bulk or of a conglomerate of firmly connected shaped bodies.

5. A composite material according to claims 1-4, made such that the shaped bodies are firmly connected chemically, physically, by fusing, glueing, sintering.

6. A composite material according to claims 1-4, made such that the material for the framework or shaped bodies can be ceramics, metals, physiologically compatible plastics, easily soluble substances such as sugar, easily fusible materials such as wax, silicones, rubber-like polymers, resorbable polymers, polylactides, polyamino acids, polyglycides, materials adapted to be extruded, in particular water-soluble materials adapted to be poured, electrolytically formed and/or processed by injection molding processes.

7. A composite material according to claim 6, made such that tricalcium phosphate, hydroxyl apatite, a mixture of both or another calcium compound adapted to be easily or hardly resorbed is used as the material for the shaped bodies.

8. A composite material according to claim 6, made such that polymers based on acrylate, polyacrylate, polymetacrylate or another physiologically compatible, resorbable or non-resorbable plastic are used as the physiologically compatible plastics.

9. A composite material according to claim 1-8, made such that the shaped bodies are ceramic and the framework is a metal casting.

10. A composite material according to claims 1-8, wherein the shaped bodies consist of an easily fusible material such as wax or sugar or another soluble, preferably water-soluble material or the shaped bodies consist of a ceramic material, preferably tricalcium phosphate, hydroxyl apatite, a mixture of both or of another calcium compound adapted to be easily or hardly resorbed.

11. A composite material according to claims 1-10, wherein the shaped bodies are substantially spherical.

12. A composite material according to one of claims 1-11, wherein the shaped bodies have a size raging between 0.2 and a few millimeters, preferably 5 mm.

13. A composite material according to one of claims 1-12, wherein a mixture of shaped bodies of varying sizes is used.

14. A composite material according to one of claims 2 and 8, wherein the shaped bodies are removed in part and the framework has a filler.

15. A composite material according to claim 14, wherein the filler consists of particles and the filler particles are 1 to 95%, preferably 1-80% tricalcium phosphate or hydroxyl apatite or a mixture of both or a calcium compound, preferably with a particle size of 50 - 300 µm, especially preferably up to 250 µm and a pore volume of 0.1 ml/g to 0.8 ml/g.

16. A composite material according to claim 15, wherein the Ca compound is a calcium phosphate compound.

17. A process for producing a composite material with the following process steps:
producing a dense package of shaped bodies or interconnecting shaped bodies to form a three-dimensional shaped body conglomerate,
shaping a material different than the shaped bodies to form a three-dimensional framework
compressing the composite body of shaped bodies and framework, removing the shaped bodies in whole or in part without destroying the framework.

18. A process according to claim 17, wherein the shaped bodies are spherical.

19. A process according to claim 17 and 18, wherein the shaped bodies are interconnected in a punctiform manner.

20. A process according to claim 17-18, wherein the shaped bodies are chemically or physically fused together or glued together, for example in steam.

21. A process according to one of claims 17-21, wherein a cast or injected-molded material is used for the three-dimensional framework and the shaped bodies are washed out, for example in a water bath or in the washing machine, after this material has hardened.

22. A process according to one of claims 17-22, wherein the shaped bodies are interconnected in a sintering process.

23. A process according to claim 17, wherein after complete removal of the shaped bodies the remaining cavity system is cast with metal.

## Revendications

1. Matériau composite constitué par une charpente tridimensionnelle formant des structures portantes à type de bâtonnets dont la dureté et l'épaisseur , la solubilité et/ou la capacité de résorption sont réglables avec exactitude, englobant les corps de formage qui se trouvent en relation par l'intermédiaire de pores interconnectés et réglables avec exactitude , la charpente tridimensionnelle réalisant un conglomérat avec les corps de formage .

2. Matériau composite selon revendication 1 , réalisé de telle façon que les corps de formage et la charpente présentent des propriétés physiques ou chimiques si différentes que les corps de formage peuvent être partiellement ou totalement enlevés sans risquer de porter atteinte à la charpente .

3. Matériau composite selon les revendications 1-2 , réalisé de façon à ce que les corps de formage constituent un paquet plus ou moins dense de billes distinctes ou en tant que conglomérat de billes composé de billes distinctes étroitement reliées entre elles .

4. Matériau composite selon les revendications 1-2 , réalisé de façon à ce que les corps de formage soient constitués par des corps uniformes géométriques , des polygones , des granulés ou par un mélange composé de corps de formage uniformes ou non uniformes et, cela, selon une distribution choisie dense ou d'un conglomérat de corps de formage étroitement interconnectés.

5. Matériau composite selon les revendications 1-4 , réalisé de façon à ce que les corps de formage soient interconnectés chimiquement , physiquement , par fusion , collage ou frittage.

6. Matériau composite selon les revendications 1-5 , réalisé de façon à ce que comme matière pour la charpente ou les corps de formage on emploie des céramiques , des métaux , des matières plastiques tolérées par l'organisme , des substances facilement solubles telles que le sucre , des matières fondant facilement telles que de la cire , des silicones , des polymères de type caoutchouc, des polymères résorbables , des polylactides , des acides polyaminés , des polyglycides , injectables , également et surtout des matériaux façonnables hydrosolubles , coulables , pouvant être traités électrolytiquement et/ou par injection de matière thermodurcissable .

7. Matériau composite selon la revendication 6 , réalisé de façon à ce que l'on emploie comme matière pour les corps de formage du phosphate tricalcique , de l'hydroxylapatite , un mélange des deux ou une autre combinaison de calcium facilement ou difficilement résorbable .

8. Matériau composite selon la revendication 6 , réalisé de façon à ce que comme matières plastiques tolérées par l'organisme l'on emploie des polymères ayant pour base un acrylate , un polyacrylate , un polymétacrylate ou une autre matière plastique résorbable ou non résorbable .

9. Matériau composite selon la revendication 1-8 , réalisé de façon à ce que les corps de formage soient constitués par de la céramique et la charpente par du métal coulé .

10. Matériau composite selon les revendications 1-8 , les corps de formage étant constitués par un matériau fondant facilement tel que la cire ou le sucre , ou par un autre matériau soluble de préférence hydrosoluble , ou compte tenu du fait que les corps de formage sont constitués par une matière céramique, de préférence du phosphate tricalcique , de l'hydroxylapatite , un mélange des deux ou d'une autre combinaison de calcium facilement ou difficilement résorbable .

11. Matériau composite selon les revendications 1-10 , les corps de formage se présentant principalement sous forme de billes .

12. Matériau composite selon les revendications 1-11 , les corps de formage présentant une grosseur allant de 0,2 à quelques mm , et s'élevant de préférence à 5 mm .

13. Matériau composite selon les revendications 1-12 , compte tenu du fait qu'on utilise un mélange de corps de formage de grosseurs différentes .

14. Matériau composite selon les revendications 2 et 8 , les corps de formage ayant été enlevés et la charpente présentant un matériau de remplissage .

15. Matériau composite selon la revendication 14 , le matériau de remplissage étant composé de particules et les particules de matériau de remplissage utilisées étant à raison de 1 à 95 % , de préférence de 1-80 %, du phosphate tricalcique ou de l'hydroxylapatite , ou bien un mélange des deux ou encore une combinaison de calcium , avec une grosseur des particules allant de 50 à 300 µm de préférence, en favorisant spécialement une grosseur allant jusqu'à 250 µm et un volume des pores de 0,1 ml/g à 0,8 ml/g .

16. Matériau composite selon la revendication 15 , la combinaison de calcium étant une combinaison de phosphate de calcium .

17. Procédé de fabrication d'un matériau composite réalisé selon les étapes suivantes :
Production d'un paquet dense de corps de formage ou combinaison de corps de formage pour réaliser un conglomérat de corps de formage tridimensionnel .
Formation d'un matériau différent de celui des corps de formage et entourant les corps de formage pour constituer une charpente tridimensionnelle .
Compactage du corps composite constitué par des corps de formage et par de la matière de la charpente . Enlèvement total ou partiel des corps de formage sans que la charpente ne soit détruite .

18. Procédé selon la revendication 17 , les corps de formage étant de forme sphérique .

19. Procédé selon les revendications 17 et 18 , les corps de formage étant interconnectés par points .

20. Procédé selon les revendications 17-18 , les corps de formage étant soudés entre eux par voie chimique ou par voie physique, ou bien étant collés solidement par exemple dans de la vapeur d'eau.

21. Procédé selon l'une des revendications 17-21 , la matière utilisée pour la charpente tridimensionnelle étant un matériau coulé ou injecté et les corps de formage étant éliminés par lavage par exemple dans un bain-marie ou dans une machine à laver .

22. Procédé selon les revendications 17-22 , les corps de formage étant interconnectés par un procédé de frittage .

23. Procédé selon la revendication 17 , le système des espaces vides subsistant après l'élimination complète des corps de formage étant comblé par du métal coulé .
